# EUROPÄISCHE PATENTSCHRIFT

(11) **EP 1 330 988 B1**
(45) Veröffentlichungstag und Bekanntmachung des Hinweises auf die Patenterteilung: **13.10.2004**
(21) Anmeldenummer: 02027461.9
(22) Anmeldetag: 10.12.2002
(51) Int. Cl.: A61B 17/72

(54) **Intramedulläres Osteosyntheseimplantat**
Intramedullary implant for osteosynthesis
Implant intramédullaire pour osteosynthèse

(30) Priorität: 18.01.2002 DE 10201743
(43) Veröffentlichungstag der Anmeldung: 30.07.2003
(73) Patentinhaber: Aesculap AG & Co. KG, 78532 Tuttlingen (DE)
(72) Erfinder: Gradl, Georg, 18211 Börgerende (DE)
(74) Vertreter: HOEGER, STELLRECHT & PARTNER Patentanwälte

(56) Entgegenhaltungen:
- WO-A-99/20195
- DE-U- 8 811 634
- US-A- 4 790 302

## Beschreibung

Die Erfindung betrifft ein intramedulläres Osteosyntheseimplantat mit einer in den Markraum eines Knochens einsetzbaren, mehrere Öffnungen zur Aufnahme von Knochenfixierelementen aufweisenden Platte.

Derartige intramedulläre Osteosyntheseimplantate werden eingesetzt, um Frakturen im Gelenk- und im metaphysären Bereich eines Röhrenknochens zu reponieren, beispielsweise am distalen Radius oder auch an der distalen Tibia. Zu diesem Zweck sind Verriegelungsnägel bekannt, die einen einseitig abgeschrägten, lösbar oder einstückig mit dem Schaft verbundenen Kopfabschnitt tragen, der ebenso wie der Schaft des Verriegelungsnagels selbst im wesentlichen kreiszylinderförmig ausgebildet ist (WO 99/20195).

Es ist auch bekannt, die Teile des zu reponierenden Röhrenknochens durch schraubenähnliche Knochenfixierelemente zu fixieren, die durch Öffnungen in einer Knochenplatte hindurchgeführt sind (winkelstabile Platte für distalen Radius der Firma Königssee Implantate und Instrumente für Osteosynthese GmbH, US-4,790,302). Dabei ist es schwierig, diese Platten am Röhrenknochen selbst festzulegen, um so eine sichere Verbindung des Röhrenknochens mit den Bruchstücken zu gewährleisten.

Es ist Aufgabe der Erfindung, ein gattungsgemäßes intramedulläres Osteosyntheseimplantat so auszubilden, daß es einerseits dazu geeignet ist, möglichst viele Knochenfixierelemente zur Festlegung der Knochenfragmente aufzunehmen und andererseits trotzdem platzsparend und positionsstabil in den jeweiligen Knochen einsetzbar zu sein.

Diese Aufgabe wird bei einem intramedullären Osteosyntheseimplantat der eingangs beschriebenen Art erfindungsgemäß dadurch gelöst, daß die Platte an einem Ende einen Schaft trägt, der mindestens eine quer zu seiner Längsachse verlaufende Verriegelungsbohrung aufweist.

Dieser Schaft kann wie ein an sich bekannter Verriegelungsnagel in den Markraum des Röhrenknochens eingesetzt und dort durch Verriegelungselemente festgelegt werden, man erhält bei dem beschriebenen Osteosyntheseimplantat also ein Bauteil, das einerseits die Eigenschaft eines Verriegelungsnagels zur Festlegung des Implantates aufweist, andererseits aber die Eigenschaften einer Knochenplatte im Bereich der zu reponierenden Knochenfragmente mit der Möglichkeit, die Knochenfragmente über eine größere Anzahl von Knochenfixierelementen festzulegen, wobei das gesamte Implantat intramedullär eingesetzt wird, die Platte sich also im Inneren des zu reponierenden Knochenfragmentbereiches befindet.

Die Platte kann gemäß einer ersten bevorzugten Ausführungsform eben ausgebildet sein, es ist aber auch möglich, daß sie gebogen ist, sei es in Längsrichtung, sei es in Querrichtung.

Die Platte weist in der Regel eine Dicke zwischen 1 mm und 5 mm auf, besonders vorteilhaft ist eine Dicke in der Größenordnung von 2 mm, wobei die Auswahl der Dicke auch von der Anatomie des zu versorgenden Röhrenknochens abhängt, hier ist dem Operateur also die Auswahl der richtigen Dicke zur optimalen Versorgung möglich.

Der Schaft kann gegenüber der Platte geneigt sein, wobei der Neigungswinkel vorzugsweise zwischen 10° und 60° liegt, eine besonders günstige Anordnung ergibt sich bei einer Neigung in der Größenordnung von 30°.

Die Neigung kann dabei in einer Ebene erfolgen, die senkrecht zur Ausdehnung der Platte verläuft, es ist aber auch möglich, daß die Neigung in einer Ebene erfolgt, die in der Ausdehnung der Platte verläuft, diese beiden Neigungen können gegebenenfalls auch kombiniert werden.

Die Platte kann um eine durch die Verbindungsstelle zwischen Schaft und Platte hindurchgehende, in der Ebene der Platte liegende Achse gegenüber dem Schaft verdreht sein.

All diese Neigungs- und Verdrehungsmöglichkeiten dienen einer optimalen Anpassung der Relativanordnung von Schaft und Platte, so daß bei einem in den Markraum eines Röhrenknochen eingesetzten Schaft die Platte entsprechend der Anatomie des zur versorgenden Knochens und entsprechend dem Verlauf der Bruchflächen optimal angeordnet werden kann.

Die Platte kann eine ovale Außenkante aufweisen, damit erhält sie eine Kontur, deren Längsausdehnung zwischen dem Schaft einerseits und dem distalen Ende des Verriegelungsnagels andererseits größer ist als die quer dazu verlaufende Breitenausdehnung. Die Kontur kann dabei direkt ovalförmig sein oder aber auch durch eine Außenkante mit geraden oder nur geringfügig gebogenen Abschnitten angenähert werden. Günstig ist es, wenn die Länge der Platte vom schaftseitigen Ende bis zum gegenüberliegenden Ende zwischen 1 cm und 15 cm liegt, besonders vorteilhaft ist eine Länge in der Größenordnung von etwa 8 cm. Auch hier ist es sinnvoll, die Länge der Anatomie der jeweils zu versorgenden Indikation anzupassen.

Die maximale Breite der Platte kann zwischen 0,5 cm und 5 cm liegen. Die Abmessungen sind dabei vorzugsweise so zu wählen, daß die Platte vollständig im Inneren des Knochens aufgenommen wird und an keiner Stelle die Knochenwand durchbricht. Je nach Größe und Form des zu versorgenden Röhrenknochens sind hier sehr unterschiedliche Abmessungen möglich, beispielsweise kann eine Abmessung von 1 cm bei einem Radius günstig sein, von 1,5 cm bei der distalen Tibia und von etwa 3 cm bei der proximalen Tibia.

Bei einer bevorzugten Ausführungsform ist vorgesehen, daß in der Platte in deren Querrichtung mindestens zwei Öffnungen für Knochenfixierelemente nebeneinander angeordnet sind, d. h. die Breite der Platte ist so gewählt, daß zumindest zwei derartige Öffnungen nebeneinander Platz finden.

Die Öffnungen können so ausgebildet sein, daß Knochenfixierungselemente im wesentlichen parallel zueinander durch sie hindurchgesteckt werden können, bei einer besonders bevorzugten Ausführungsform ist jedoch vorgesehen, daß die Längsachsen von mehreren als zylindrische Bohrungen ausgebildeten Öffnungen relativ zueinander geneigt sind. Dadurch werden die eingesetzten Knochenfixierungselemente zwangsweise in einer gegeneinander geneigten Position im Knochen gehalten und können so die einzelnen Knochenfragmente besonders wirksam stabilisieren.

Beispielsweise können die Längsachsen der Öffnungen derart geneigt sein, daß die Spitzen der durch diese Öffnungen ragenden Knochenfixierelemente konvergieren.

Es ist dabei vorteilhaft, wenn die Längsachsen der Öffnungen so gegeneinander geneigt sind, daß sich in die Öffnungen eingesetzte Knochenfixierelemente in einer quer zur Längsachse des Schaftes verlaufenden Projektionsebene überkreuzen. Genauer gesagt überkreuzen sich dabei die Projektionen der Knochenfixierungselemente in dieser Projektionsebene.

Es ist weiterhin vorteilhaft, wenn die Längsachsen der Öffnungen so gegeneinander geneigt sind, daß in die Öffnungen eingesetzten Knochenfixierelemente in einer durch die Längsachse des Schaftes definierten Projektionsebene ohne Überkreuzung konvergieren. Die Konvergenz ist also nur so groß, daß keine Überkreuzung der Knochenfixierelemente eintritt.

Bei einer abgewandelten Ausführungsform sind mindestens zwei Öffnungen vorgesehen, deren Längsachsen in einer quer zur Längsachse des Schaftes verlaufenden Projektionsebene divergieren, in diese Öffnungen eingesetzte Knochenfixierelemente überkreuzen sich also nicht.

Die Knochenfixierelemente können einfache Stifte sein, vorteilhafterweise handelt es sich dabei jedoch um Knochenschrauben, insbesondere um selbstschneidende Knochenschrauben.

Günstig ist es dabei, wenn die als Knochenschrauben ausgebildeten Knochenfixierelemente im Bereich eines Schraubenkopfes ein größeres Gewinde aufweisen als im übrigen Bereich, größer kann dabei sowohl im Hinblick auf den Durchmesser als auch im Hinblick auf die Geometrie der Schraubengänge verstanden werden, so daß eine derartige Knochenschraube mit ihrem kopfnahen Bereich im voll eingeschraubten Zustand sicher und in ihrer Winkellage fixiert in der Außenwand des Knochens gehalten wird. Dadurch ergibt sich eine besonders gute Stabilität der reponierten Knochenfragmente.

Die Öffnungen können ein Innengewinde tragen, auch dies trägt zur Stabilisierung bei.

Der Schaft kann bei einer ersten bevorzugten Ausführungsform geradlinig ausgebildet sein, es ist aber auch möglich, daß der Schaft zumindest bereichsweise gebogen ist, um dadurch eine optimale Anpassung an die Geometrie des Knochenhohlraumes zu erreichen.

Bei einer bevorzugten Ausführungsform ist vorgesehen, daß die Platte an ihrem dem Schaft gegenüberliegenden Ende einen Angriffsbereich für ein Handhabungswerkzeug aufweist. Das Handhabungswerkzeug kann in diesem Angriffsbereich fest mit der Platte verbunden werden, so daß das Osteosyntheseimplantat praktisch mit einem Handgriff oder einem Einschlaginstrument verbunden ist, mit dessen Hilfe das Osteosyntheseimplantat in die gewünschte Position im Knochen gebracht werden kann.

Besonders vorteilhaft ist es , wenn das Handhabungswerkzeug eine an dem Angriffsbereich anlegbare und diesen klemmend umgebende Spannzange umfaßt. Es ist dann möglich, das Handhabungswerkzeug durch Spannen der Spannzange fest mit dem Osteosyntheseimplantat zu verbinden, diese Verbindung kann einfach durch Lösen der Spannzange wieder aufgehoben werden.

Insbesondere kann vorgesehen sein, daß das Handhabungswerkzeug mit einer Bohrlehre verbunden ist, die bei an dem Angriffsbereich anliegendem Handhabungswerkzeug Öffnungen für ein Bohrwerkzeug aufweist, die mit den Öffnungen in der Platte und/oder dem Schaft ausgerichtet sind. Man erhält dann also eine Baueinheit aus dem Osteosyntheseimplantat, dem Handhabungswerkzeug und der Bohrlehre, diese Baueinheit umfaßt starr miteinander verbundene Bauteile und ermöglicht es dem Operateur nicht nur, das Osteosymtheseimplantat in die gewünschte Position zu bringen, sondern stellt auch gleichzeitig sicher, daß der Operateur mit Hilfe der Bohrlehre Bohrungen in den Knochen einbringen kann, die mit den Öffnungen in der Platte und/oder den Öffnungen im Schaft ausgerichtet sind. Durch Lösen der Verbindung zwischen Handhabungswerkzeug und Osteosyntheseimplantat kann dann wieder eine Trennung erfolgen.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines sehr schematisch dargestellten Röhrenknochens mit einem eine Platte und einen Schaft umfassenden Osteosyntheseimplantat mit einer größeren Anzahl von die Platte durchsetzenden Knochenschrauben;
- Figur 2:: eine Draufsicht auf den Knochen der Figur 1;
- Figur 3:: eine Seitenansicht eines intramedullären Osteosynthese-implantats mit einem geradlinigen Schaft und einer ebenen Platte in Seitenansicht;
- Figur 4:: eine Vorderansicht des Osteosyntheseimplantates der Figur 3 mit eingesetzten, konvergierenden Knochenschrauben;
- Figur 5:: eine Ansicht der Platte des Osteosyntheseimplantates der Figur 4 mit in seitlicher Richtung divergierenden und in Längsrichtung konvergierenden Knochenschrauben;
- Figur 6:: ein abgewandeltes Ausführungsbeispiel eines intramedullären Osteosyntheseimplantates mit gebogenem Schaft und parallel zueinander verlaufenden Öffnungen für Knochenfixierelemente in der Platte;
- Figur 7:: eine Draufsicht auf das Osteosyntheseimplantat der Figur 6;
- Figur 8:: eine Ansicht ähnlich Figur 3 mit einer seitlich geneigten Platte;
- Figur 9:: eine Draufsicht auf das Osteosyntheseimplantat der Figur 8;
- Figur 10:: eine Ansicht ähnlich Figur 8 mit einer um ihre Längsachse verdrehten Platte;
- Figur 11:: eine Draufsicht auf das Osteosyntheseimplantat der Figur 10;
- Figur 12:: eine schematische Seitenansicht eines in einen Röhrenknochen eingesetzten Osteosyntheseimplantats und eines mit diesem verbundenen Handhabungswerkzeuges;
- Figur 13:: eine Vorderansicht des oberen Teils des Handhabungswerkzeuges der Figur 12 und
- Figur 14:: eine vergrößerte Längsschnittansicht des Handhabungswerkzeugs der Figur 12 im Verbindungsbereich mit der Platte des Osteosyntheseimplantates.

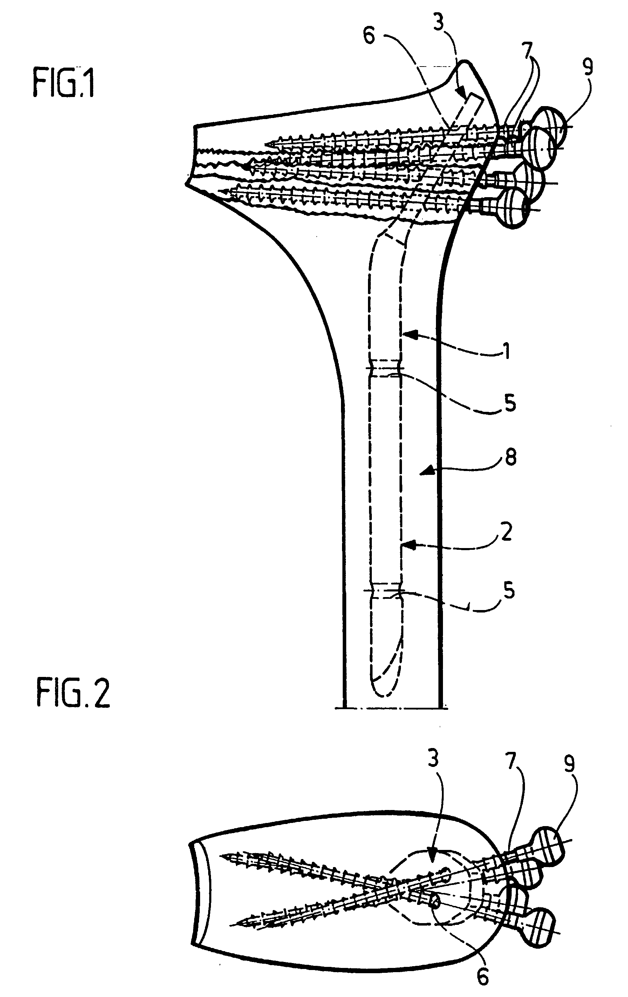

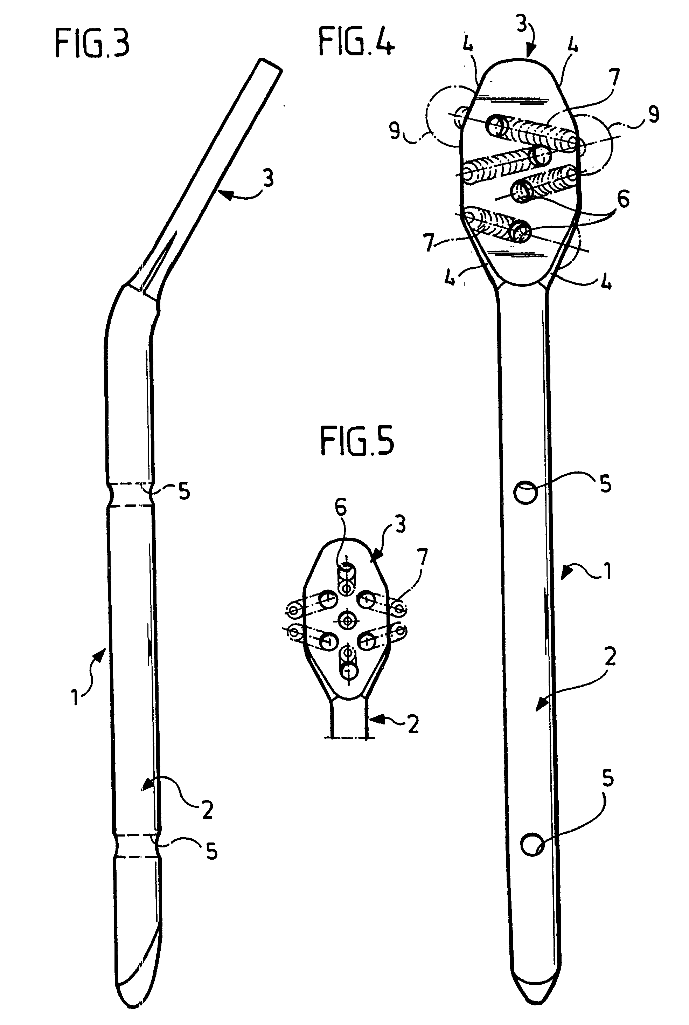

Das in den Figuren 1 bis 5 dargestellte intramedulläre Osteosyntheseimplantat, im folgenden kurz Implantat 1 genannt, umfaßt einen geradlinigen Schaft 2 mit kreisförmigem Querschnitt mit einer Länge zwischen 1 cm und 30 cm, wobei diese Länge an die jeweiligen anatomischen Gegebenheiten angepaßt wird, und mit einer sich an diesen Schaft 2 anschließenden flachen, ebenen Platte 3, die gegenüber der Längsachse des Schaftes 2 in einer senkrecht zur Ebene der Platte 3 verlaufenden Ebene um etwa 30° abgewinkelt ist. Die Platte 3 weist eine Breite auf, die im dargestellten Ausführungsbeispiel etwa dem dreifachen Durchmesser des Schaftes 2 entspricht, und eine Länge, die deutlich größer ist als die Breite, beispielsweise ist die Platte 3 doppelt so lang wie breit. Die Außenkontur ist durch Abschrägungen 4 an den gegenüberliegenden Enden der Platte 3 etwa oval ausgebildet, wobei der ovale Verlauf durch kürzere geradlinige Begrenzungslinien angenähert wird (Figur 4).

Der Durchmesser des Schaftes 2 liegt etwa zwischen 2 mm und 12 mm und die Dicke der Platte zwischen 1 mm und 5 mm. Dabei sind diese Abmessungen vorzugsweise individuell an die Anatomie der jeweils zu versorgenden Indikation und den daraus abzuleitenden biomechanischen Belastungen angepaßt. Beispielsweise kann der Durchmesser des Schaftes beim Radius etwa bei 4 mm liegen, bei der distalen Tibia etwa bei 6 mm oder bei der proximalen Tibia bei 10 mm, die Dicke der Platte kann beim Radius bei 2 mm liegen, bei der distalen Tibia bei 3 mm oder bei der proximalen Tibia bei 4 mm. Selbstverständlich sind auch diese Abmessungen entsprechend den individuellen Unterschieden veränderbar.

Schaft und Platte sind vorzugsweise einstückig ausgebildet, es ist aber auch möglich, Schaft und Platte als voneinander unabhängige Bauteile auszubilden, die miteinander verbunden werden. In diesem Falle ist es möglich, Schäfte und Platten unterschiedlicher Abmessungen und Formgebungen je nach Bedarf miteinander zu kombinieren. Insbesondere können Bausätze mit unterschiedlichen Schaftgeometrien und unterschiedlichen Plattengeometrien verwendet werden, aus denen der Operateur jeweils die notwendigen Einzelteile auswählt und dann zu dem gewünschten intramedullären Osteosyntheseimplantat kombiniert.

Das Osteosyntheseimplantat besteht normalerweise aus den üblichen metallischen Werkstoffen, beispielsweise aus Titan oder einer Titanlegierung, es können aber auch Kunststoffe zum Einsatz kommen, beispielsweise kann das Osteosyntheseimplantat aus Polyetheretherketon bestehen oder anderen bioverträglichen Kunststoffen, insbesondere aus resorbierbaren Kunststoffen.

Im Schaft 2 sind zwei diesen quer durchsetzende Verriegelungsbohrungen 5 im Abstand zueinander angeordnet, in der Platte 3 befinden sich eine größere Anzahl von Bohrungen 6, die zumindest teilweise als Innengewindebohrung ausgebildet sind. Diese Bohrungen 6 sind über die Fläche der Platte sowohl in Längsrichtung als auch in Querrichtung verteilt und die Längsachsen der Bohrungen 6 sind bei dem in den Figuren 1 bis 5 dargestellten Ausführungsbeispiel gegeneinander geneigt. In einer Längsebene, in der die Längsachse des Schaftes 2 verläuft, konvergieren die Längsachsen der Bohrung 6 dabei so, daß in die Bohrungen 6 eingeführte Knochenschrauben 7 zwar aufeinander zulaufen, sich aber nicht überkreuzen und auch nicht berühren (Figur 1).

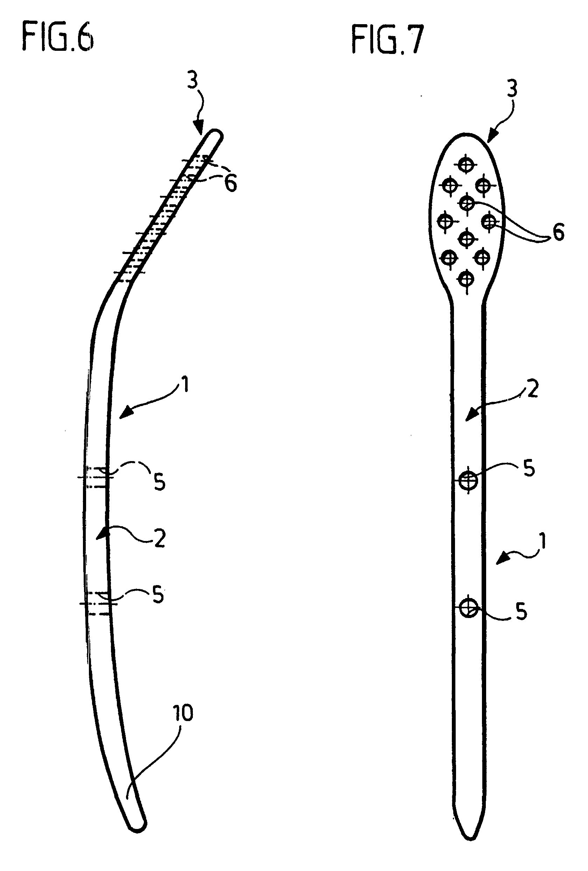

In einer quer zur Längsachse des Schaftes 2 verlaufenden Ebene können die Bohrungen 6 stärker konvergieren, so daß in die Bohrungen eingesetzte Knochenschrauben 7 sich in dieser Ebene gesehen überkreuzen (Figur 2). Es ist aber auch möglich, daß die Längsachsen von Bohrungen 6 divergieren, so daß durch sie hindurchgesteckte Knochenschrauben 7 in der quer zur Längsachse des Schaftes 2 verlaufenden Querebene divergieren (Figur 5). Bei diesem Ausführungsbeispiel der Figur 5 ist also die divergierende Anordnung in der Querebene kombiniert mit einer konvergierenden Anordnung in der Längsebene.

Das Implantat 1 wird mit seinem Schaft 2 in den Markraum eines Röhrenknochens 8 so weit eingeführt, bis die Platte 3 vollständig im Inneren des Knochens angeordnet ist, und zwar im Bereich des Kopfes dieses Knochens. Nach dem Einsetzen kann das Implantat 1 im Röhrenknochen 8 durch in der Zeichnung nicht dargestellte Verriegelungsschrauben festgelegt werden, die von der Außenseite des Röhrenknochens 8 ausgehend durch die Knochenwand und anschließend durch die Verriegelungsbohrungen 5 des Schaftes 2 hindurchgeschraubt werden. An der Platte 3 können durch Knochenschrauben 7 die zu reponierenden und gegenüber dem Röhrenknochen 8 zu fixierenden Knochenfragmente festgelegt werden, dazu werden die Knochenschrauben 7 durch die Knochenfragmente und die Bohrungen 6 hindurchgeschraubt. Durch die größere Anzahl von Bohrungen 6 und deren unterschiedlicher Orientierung ergeben sich für den Operateur eine Vielzahl von Positioniermöglichkeiten für Knochenschrauben, so daß eine optimale Anpassung an die unterschiedlichen Geometrien der Frakturteile möglich ist. Vorzugsweise haben die Knochenschrauben 7 angrenzend an ihre Schraubenköpfe 9 einen Gewindebereich mit größeren Abmessungen, sei es durch größeren Durchmesser, sei es durch größere Abmessungen der Schraubengänge, so daß die eingeschraubte Knochenschraube 7 durch diesen vergrößerten Gewindebereich in der stabilen Außenwand des Röhrenknochens 8 sicher festgelegt und winkelmäßig fixiert wird. Dadurch ergibt sich eine Fixierung der Knochenschraube einmal durch die Knochenaußenwand und zum anderen durch die Platte 3 im Inneren des Knochens, diese Fixierung ist insbesondere dann besonders wirksam, wenn die Bohrung 6 als Innengewindebohrung ausgebildet ist.

Bei dieser Ausgestaltung wird die Vielseitigkeit, die bei der Osteosynthese durch Knochenplatten ermöglicht wird, kombiniert mit der sicheren Festlegung der Platte im Inneren des Knochens durch den Schaft 2, man erhält also ein Implantat 1, das die Vorteile einer Knochenplatte und eines Verriegelungsnagels verbindet. Besonders günstig ist bei dieser Ausgestaltung, daß die Knochenplatte im Inneren des Knochens positioniert werden kann und damit Irritationen des umgebenden Weichteilgewebes vermieden werden, die bei Verwendung von äußeren Knochenplatten nicht zu verhindern sind.

Das in den Figuren 6 und 7 dargestellte Ausführungsbeispiel ist ähnlich aufgebaut wie das der Figuren 1 bis 5, einander entsprechende Teile tragen daher die gleichen Bezugszeichen.

Bei dem Ausführungsbeispiel der Figur 6 sind die Bohrungen 6 parallel zueinander ausgerichtet, wobei die Längsrichtung etwa senkrecht zur Längsachse des Schaftes 2 verläuft, also gegenüber der geneigten Platte 3 um einen Winkel geneigt, der etwa dem Neigungswinkel der Platte gegenüber dem Schaft entspricht. Außerdem ist die Außenkontur der Platte 3 in diesem Ausführungsbeispiel oval ausgebildet, d. h. nicht lediglich durch geradlinige Abschnitte an ein Oval angenähert, sondern genau oval geformt.

Schließlich ist an diesem Ausführungsbeispiel der Schaft 2 nicht geradlinig ausgebildet, sondern in seinem distalen Bereich abgebogen, diese Abbiegung 10 verläuft aber in derselben Richtung wie die Neigung der Platte 3.

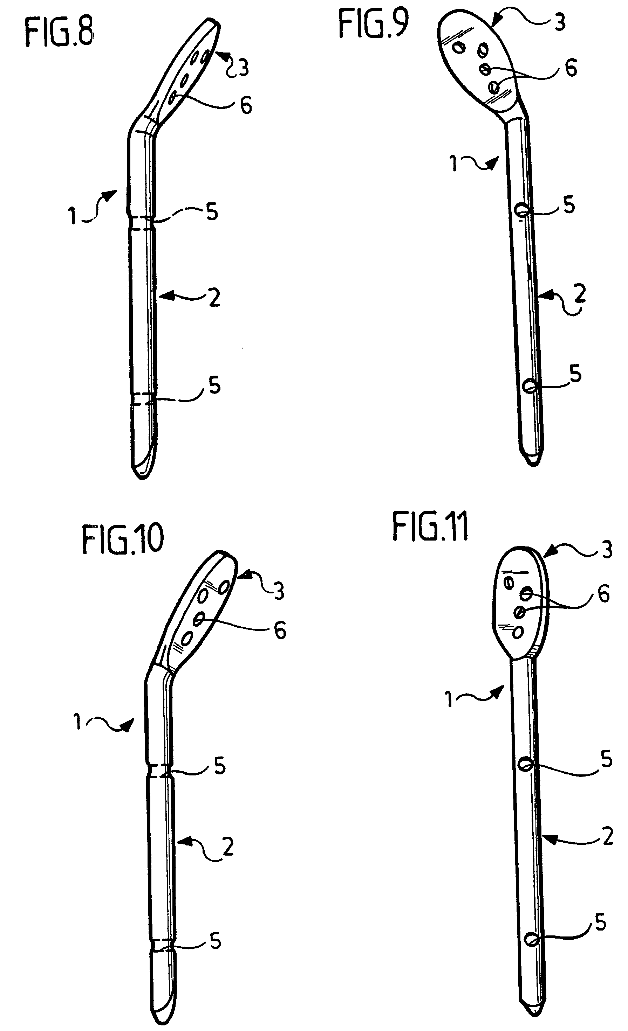

Das in den Figuren 8 und 9 dargestellte Ausführungsbeispiel eines Osteosyntheseimplantates entspricht weitgehend dem der Figuren 3 und 4, einander entsprechende Teile tragen daher dieselben Bezugszeichen. Bei diesem Ausführungsbeispiel ist die Platte 3 gegenüber dem Schaft 2 nicht nur in einer Ebene geneigt, die senkrecht auf der Fläche der Platte 3 steht, sondern zusätzlich auch in der Ebene der Platte 3.

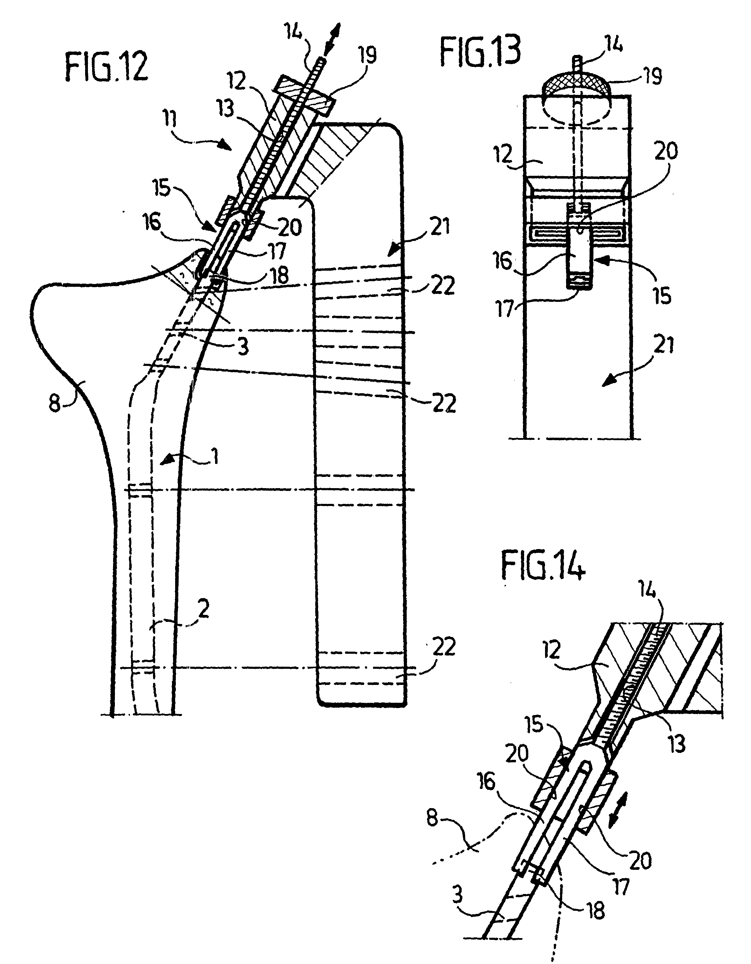

Beim Ausführungsbeispiel der Figuren 10 und 11, das wiederum dem der Figuren 3 und 4 ähnlich ist und daher mit denselben Bezugszeichen gekennzeichnet ist, ist die Platte 3 um ihre Längsachse relativ zum Schaft 2 verdreht, also um eine Achse, die durch die Verbindungsstelle zwischen Schaft 2 und Platte 3 verläuft und die Längsmittelachse der Platte 3 beschreibt.

Die in den Ausführungsbeispielen gezelgten unterschiedlichen Neigungen und Winkelstellungen der Platte 3 relativ zum Schaft 2 können miteinander kombiniert werden, so daß insgesamt die Platte 3 gegenüber dem Schaft 2 eine beliebige Position einnehmen kann, die vom Operateur je nach den anatomischen Gegebenheiten ausgewählt wird.

Alle vorbeschriebenen Merkmale des Implantates 1 können gemeinsam oder auch nur einzeln verwirklicht werden, es ist auch möglich, die Ausgestaltungen aller Ausführungsbeispiele miteinander zu kombinieren, beispielsweise die Schaftform, die Plattenform, die relative Anordnung von Platte und Schaft und die unterschiedlichen Abmessungen von Platte und Schaft. Der Schaft kann dabei einen beliebigen Querschnitt aufweisen, hat also beispielsweise einen kreisförmigen, ovalen, dreiflächigen oder vierflächigen Querschnitt mit abgerundeten Kanten.

Um das Implantat 1 im Röhrenknochen in die gewünschte Position bringen zu können, kann ein Handhabungswerkzeug 11 benutzt werden, wie es in den Figuren 12 bis 14 dargestellt ist. Dieses Handhabungswerkzeug 11 umfaßt ein längliches Gehäuse 12 mit einer durchgehenden Bohrung 13, durch die eine Gewindespindel 14 hindurchgeführt ist. Diese trägt an einem Ende eine spannzangenähnliche Gabel 15 mit zwei parallelen Zinken 16, 17, die am oberen Ende der Platte 3, also an dem dem Schaft 2 gegenüberliegenden Ende der Platte 3, an gegenüberliegenden Seiten an die Platte 3 anlegbar sind, so daß die Platte 3 in einem zwischen den Zinken 16, 17 liegenden Anlagebereich 18 zwischen den Zinken 16 und 17 aufgenommen ist. Zum Festspannen der Zinken 16, 17 ist die Spannzange 15 mittels der Gewindespindel 14 in das Gehäuse 12 einziehbar, und zwar durch eine sich am Gehäuse 12 abstützende, auf die Gewindespindel 14 aufgeschraubte Stellmutter 19, und dabei legen sich die Außenseiten der Zinken 16, 17 in zunehmendem Maße an Spannflächen 20 des Gehäuses 12 an, die die Zinken 16, 17 beim Einschieben der Spannzange 15 zwischen die Spannflächen 20 zusammendrücken. Auf diese Weise ist es möglich, eine starre Klemmverbindung zwischen Handhabungswerkzeug 11 und Implantat 1 herzustellen.

An dem Gehäuse 12 ist seitlich eine Bohrlehre 21 gehalten, die sich im wesentlichen parallel zum Implantat 1 erstreckt, wenn dieses in der Spannzange 15 gehalten ist, und zwar derart, daß Öffnungen 22 in der Bohrlehre 21 mit den Bohrungen 6 in der Platte 3 und mit den Verriegelungsbohrungen 5 im Schaft 2 ausgerichtet sind. Damit ist es möglich, unter Verwendung der Bohrlehre 21 in den Röhrenknochen 8 Bohrungen einzubringen, die ebenfalls mit den Verriegelungsbohrungen 5 und den Bohrungen 6 ausgerichtet sind und damit ein Einsetzen von Knochenschrauben 7 ermöglichen.

Die Darstellung der Figuren 12 bis 14 ist schematisch erfolgt, es versteht sich, daß die Spannzange 15 an dem Anlagebereich 18 der Platte 3 in genau definierter Position anzulegen ist, um eine Ausrichtung der Öffnungen 22 mit den Bohrungen 6 und den Verriegelungsbohrungen 5 zu erreichen, dies kann durch geeignete Anschläge oder durch einen Formschluß zwischen den Teilen des Handhabungswerkzeuges 11 einerseits und der Platte 3 andererseits erreicht werden, dies ist jedoch in der Zeichnung nicht dargestellt.

## Patentansprüche

1. Intramedulläres Osteosyntheseimplantat (1) mit einer in den Markraum eines Knochens (8) einsetzbaren, mehrere Öffnungen (22) zur Aufnahme von Knochenfixierelementen (7) aufweisenden Platte (3), **dadurch gekennzeichnet, daß** die Platte (3) an einem Ende einen Schaft (2) trägt, der mindestens eine quer zu seiner Längsachse verlaufende Verriegelungsbohrung (5) aufweist.

2. Osteosyntheseimplantat nach Anspruch 1, **dadurch gekennzeichnet, daß** die Platte (3) eben ist.

3. Osteosyntheseimplantat nach Anspruch 1 oder 2, **dadurch gekennzeichnet, daß** die Platte (3) eine Dicke zwischen 1 mm und 5 mm aufweist.

4. Osteosyntheseimplantat nach einem der voranstehenden Ansprüche, **dadurch gekennzeichnet, daß** der Schaft (2) gegenüber der Platte (3) geneigt ist.

5. Osteosyntheseimplantat nach Anspruch 4, **dadurch gekennzeichnet, daß** der Neigungswinkel zwischen 15° und 60° liegt.

6. Osteosyntheseimplantat nach Anspruch 4 oder 5, **dadurch gekennzeichnet, daß** die Neigung in einer Ebene erfolgt, die senkrecht zur Ausdehnung der Platte (3) verläuft.

7. Osteosyntheseimplantat nach einem der Ansprüche 4 bis 6, **dadurch gekennzeichnet, daß** die Neigung in einer Ebene erfolgt, die in der Ausdehnung der Platte (3) verläuft.

8. Osteosyntheseimplantat nach einem der voranstehenden Ansprüche, **dadurch gekennzeichnet, daß** die Platte (3) um eine durch die Verbindungsstelle zwischen Schaft (2) und Platte (3) hindurchgehende, in der Ebene der Platte (3) liegende Achse gegenüber dem Schaft (2) verdreht ist.

9. Osteosyntheseimplantat nach einem der voranstehenden Ansprüche, **dadurch gekennzeichnet, daß** die Platte (3) eine ovale Außenkante aufweist.

10. Osteosyntheseimplantat nach einem der voranstehenden Ansprüche, **dadurch gekennzeichnet, daß** die Länge der Platte (3) vom schaftseitigen Ende bis zum gegenüberliegenden Ende der Platte (3) zwischen 1 cm und 15 cm liegt.

11. Osteosyntheseimplantat nach einem der voranstehenden Ansprüche, **dadurch gekennzeichnet, daß** die maximale Breite der Platte (3) zwischen 0,5 cm und 5 cm liegt.

12. Osteosyntheseimplantat nach einem der voranstehenden Ansprüche, **dadurch gekennzeichnet, daß** in der Platte (3) in deren Querrichtung mindestens zwei Öffnungen (6) für Knochenfixierelemente (7) nebeneinander angeordnet sind.

13. Osteosyntheseimplantat nach einem der voranstehenden Ansprüche, **dadurch gekennzeichnet, daß** die Längsachsen von mehreren als zylindrische Bohrungen ausgebildete Öffnungen (6) relativ zueinander geneigt sind.

14. Osteosyntheseimplantat nach Anspruch 13, **dadurch gekennzeichnet, daß** die Längsachsen der Öffnungen (6) derart geneigt sind, daß die Spitzen der durch diese Öffnungen (6) ragenden Knochenfixierelemente (7) konvergieren.

15. Osteosyntheseimplantat nach Anspruch 14, **dadurch gekennzeichnet, daß** die Längsachsen der Öffnungen (6) so gegeneinander geneigt sind, daß sich in die Öffnungen (6) eingesetzte Knochenfixierelemente (7) in einer quer zur Längsachse des Schaftes (2) verlaufenden Projektionsebene überkreuzen.

16. Osteosyntheseimplantat nach Anspruch 14 oder 15, **dadurch gekennzeichnet, daß** die Längsachsen der Öffnungen (6) so gegeneinander geneigt sind, daß in die Öffnungen (6) eingesetzte Knochenfixierelemente (7) in einer durch die Längsachse des Schaftes (2) definierten Projektionsebene ohne Überkreuzung konvergieren.

17. Osteosyntheseimplantat nach Anspruch 13, **dadurch gekennzeichnet, daß** mindestens zwei Öffnungen (6) vorgesehen sind, deren Längsachsen in einer quer zur Längsachse des Schaftes (2) verlaufenden Projektionsebene divergieren.

18. Osteosyntheseimplantat nach einem der voranstehenden Ansprüche, **dadurch gekennzeichnet, daß** die Knochenfixierelemente (7) Knochenschrauben sind.

19. Osteosyntheseimplantat nach Anspruch 18, **dadurch gekennzeichnet, daß** die Knochenfixierelemente (7) im Bereich eines Schraubenkopfes (9) ein größeres Gewinde aufweisen als im übrigen Bereich.

20. Osteosyntheseimplantat nach einem der voranstehenden Ansprüche, **dadurch gekennzeichnet, daß** die Öffnungen (6) ein Innengewinde tragen.

21. Osteosyntheseimplantat nach einem der voranstehenden Ansprüche, **dadurch gekennzeichnet, daß** der Schaft (2) geradlinig ausgebildet ist.

22. Osteosyntheseimplantat nach einem der Ansprüche 1 bis 20, **dadurch gekennzeichnet, daß** der Schaft (2) zumindest bereichsweise gebogen ist.

23. Osteosyntheseimplantat nach Anspruch 22, **dadurch gekennzeichnet, daß** die Abbiegung (10) des Schaftes (2) und eine Abwinklung der Platte (3) in dieselbe Richtung verlaufen.

24. Osteosyntheseimplantat nach einem der voranstehenden Ansprüche, **dadurch gekennzeichnet, daß** die Platte (3) an ihrem dem Schaft (2) gegenüberliegenden Ende einen Angriffsbereich (18) für ein Handhabungswerkzeug (11) aufweist.

25. Osteosyntheseimplantat nach Anspruch 24, **dadurch gekennzeichnet, daß** das Handhabungswerkzeug (11) eine an dem Angriffbereich (18) anlegbare und diesen klemmend umgebende Spannzange (15) umfaßt.

26. Osteosyntheseimplantat nach einem der Ansprüche 23 oder 24, **dadurch gekennzeichnet, daß** das Handhabungswerkzeug (11) mit einer Bohrlehre (21) verbunden ist, die bei an dem Angriffsbereich (18) anliegendem Handhabungswerkzeug (11) Öffnungen (22) für ein Bohrwerkzeug aufweist, die mit den Öffnungen (6; 5) in der Platte (3) und/oder dem Schaft (2) ausgerichtet sind.

## Claims

1. An intramedullary osteo-synthetic implant (1) comprising a plate (3) insertable into the medullary space of a bone (8) and having several openings (22) [sic, recte 6] to receive bone fixation elements (7), **characterised in that** the plate (3) bears a shaft (2) at one end which has at least one locking bore (5) extending transversely to its longitudinal axis.

2. An osteo-synthetic implant according to Claim 1, **characterised in that** the plate (3) is flat.

3. An osteo-synthetic implant according to Claim 1 or 2, **characterised in that** the plate (3) has a thickness between 1 mm and 5 mm.

4. An osteo-synthetic implant according to one of the preceding Claims, **characterised in that** the shaft (2) is inclined in relation to the plate (3).

5. An osteo-synthetic implant according to Claim 4, **characterised in that** the angle of inclination is between 15° and 60°.

6. An osteo-synthetic implant according to Claim 4 or 5, **characterised in that** the inclination occurs in a plane which extends perpendicularly to the extent of the plate (3).

7. An osteo-synthetic implant according to one of Claims 4 to 6, **characterised in that** the inclination occurs in a plane which extends in the extent of the plate (3).

8. An osteo-synthetic implant according to one of the preceding claims, **characterised in that** the plate (3) is rotated relative to the shaft (2) around an axis passing through the junction point between the shaft (2) and the plate (3) and lying in the plane of the plate (3).

9. An osteo-synthetic implant according to one of the preceding claims, **characterised in that** the plate (3) has an oval outside edge.

10. An osteo-synthetic implant according to one of the preceding claims, **characterised in that** the length of the plate (3) from the end on the shaft side to the opposite end of the plate (3) lies between 1 cm and 15 cm.

11. An osteo-synthetic implant according to one of the preceding claims, **characterised in that** the maximum width of the plate (3) lies between 0.5 cm and 5 cm.

12. An osteo-synthetic implant according to one of the preceding claims, **characterised in that** at least two openings (6) for bone fixation elements (7) are arranged adjacent to one another in the plate (3) in the transverse direction of the said plate.

13. An osteo-s ynthetic implant according to one of the preceding claims, **characterised in that** the longitudinal axes of several openings (6) in the form of cylindrical bores are inclined relative to one another.

14. An osteo-synthetic implant according to Claim 13, **characterised in that** the longitudinal axes of the openings (6) are inclined in such a way that the tips of the bone fixation elements (7) projecting through these openings (6) converge.

15. An osteo-synthetic implant according to Claim 14, **characterised in that** the longitudinal axes of the openings (6) are inclined relative to one another such that bone fixation elements (7) inserted into the openings (6) cross over one another in a projection plane extending transversely to the longitudinal axis of the shaft (2).

16. An osteo-synthetic implant according to Claim 14 or 15, **characterised in that** the longitudinal axes of the openings (6) are inclined relative to one another such that bone fixation elements (7) inserted into the openings (6) converge without crossing in a projection plane defined by the longitudinal axis of the shaft (2).

17. An osteo-synthetic implant according to Claim 13, **characterised in that** at least two openings (6) are provided, the longitudinal axes of which diverge in a projection plane extending transversely to the longitudinal axis of the shaft (2).

18. An osteo-synthetic implant according to one of the preceding claims, **characterised in that** the bone fixation elements (7) are bone screws.

19. An osteo-synthetic implant according to Claim 18, **characterised in that** the bone fixation elements (7) have a larger thread in the region of a screw head (9) than in the remaining region.

20. An osteo-synthetic implant according to one of the preceding claims, **characterised in that** the openings (6) bear an internal thread.

21. An osteo-synthetic implant according to one of the preceding claims, **characterised in that** the shaft (2) is designed so as to be straight.

22. An osteo-synthetic implant according to one of Claims 1 to 20, **characterised in that** the shaft (2) is curved at least in regions.

23. An osteo-synthetic implant according to Claim 22, **characterised in that** the curvature (10) of the shaft (2) and angling of the plate (3) extend in the same direction.

24. An osteo-synthetic implant according to one of the preceding claims, **characterised in that** the plate (3) has at its end opposite the shaft (2) an application region (18) for a manipulation instrument (11).

25. An osteo-synthetic implant according to Claim 24, **characterised in that** the manipulation instrument (11) comprises gripping tongs (15) which may be applied to the application region (18) and enclose the said region so as to effect clamping.

26. An osteo-synthetic implant according to one of Claims 23 or 24, **characterised in that** the manipulation instrument (11) is connected to a drilling jig (21) which, with the manipulation instrument (11) applied to the application region (18), has openings (22) for a drilling tool, the said openings being aligned with the openings (6; 5) in the plate (3) and/or the shaft (2).

## Revendications

1. Implant intramédullaire pour ostéosynthèse (1) avec une plaque (3) à insérer dans le canal médullaire d'un os (8) et présentant plusieurs ouvertures (22) pour recevoir des éléments de fixation d'os (7), **caractérisé en ce que** la plaque (3) porte à une extrémité une tige (2) qui présente au moins un perçage de verrouillage (5) s'étendant transversalement à son axe longitudinal.

2. Implant pour ostéosynthèse selon la revendication 1, **caractérisé en ce que** la plaque (3) est plane.

3. Implant pour ostéosynthèse selon la revendication 1 ou 2, **caractérisé en ce que** la plaque (3) présente une épaisseur entre 1 mm et 5 mm.

4. Implant pour ostéosynthèse selon l'une quelconque des revendications précédentes, **caractérisé en ce que** la tige (2) est inclinée par rapport à la plaque (3).

5. Implant pour ostéosynthèse selon la revendication 4, **caractérisé en ce que** l'angle d'inclinaison se situe entre 15° et 60°.

6. Implant pour ostéosynthèse selon la revendication 4 ou 5, **caractérisé en ce que** l'inclinaison a lieu dans un plan qui s'étend perpendiculairement à l'extension de la plaque (3).

7. Implant pour ostéosynthèse selon l'une quelconque des revendications 4 à 6, **caractérisé en ce que** l'inclinaison a lieu dans un plan qui s'étend dans l'extension de la plaque (3).

8. Implant pour ostéosynthèse selon l'une quelconque des revendications précédentes, **caractérisé en ce que** la plaque (3) est déformée par rapport à la tige (2) autour d'un axe traversant la jonction entre la tige (2) et la plaque (3) et situé dans le plan de la plaque (3).

9. Implant pour ostéosynthèse selon l'une quelconque des revendications précédentes, **caractérisé en ce que** la plaque (3) présente un bord extérieur ovale.

10. Implant pour ostéosynthèse selon l'une quelconque des revendications précédentes, **caractérisé en ce que** la longueur de la plaque (3) de l'extrémité côté tige à l'extrémité opposée de la plaque (3) se situe entre 1 cm et 15 cm.

11. Implant pour ostéosynthèse selon l'une quelconque des revendications précédentes, **caractérisé en ce que** la largeur maximale de la plaque (3) se situe entre 0,5 cm et 5 cm.

12. Implant pour ostéosynthèse selon l'une quelconque des revendications précédentes, **caractérisé en ce que** dans la plaque (3), dans sa direction transversale, au moins deux ouvertures (6) pour des éléments de fixation d'os (7) sont disposés côte à côte.

13. Implant pour ostéosynthèse selon l'une quelconque des revendications précédentes, **caractérisé en ce que** les axes longitudinaux de plusieurs ouvertures (6) réalisées sous forme de perçages cylindriques sont inclinés les uns par rapport aux autres.

14. Implant pour ostéosynthèse selon la revendication 13, **caractérisé en ce que** les axes longitudinaux des ouvertures (6) sont inclinés de telle sorte que les pointes des éléments de fixation d'os (7) dépassant à travers ces ouvertures (6) convergent.

15. Implant pour ostéosynthèse selon la revendication 14, **caractérisé en ce que** les axes longitudinaux des ouvertures (6) sont inclinés les uns par rapport aux autres de telle sorte que des éléments de fixation d'os (7) insérés dans les ouvertures (6) se croisent dans un plan de projection s'étendant transversalement à l'axe longitudinal de la tige (2).

16. Implant pour ostéosynthèse selon la revendication 14 ou 15, **caractérisé en ce que** les axes longitudinaux des ouvertures (6) sont inclinés les uns par rapport aux autres de telle sorte que des éléments de fixation d'os (7) insérés dans les ouvertures (6) convergent dans un plan de projection défini par l'axe longitudinal de la tige (2) sans se croiser.

17. Implant pour ostéosynthèse selon la revendication 13, **caractérisé en ce qu'**au moins deux ouvertures (6) sont prévues dont les axes longitudinaux divergent dans un plan de projection s'étendant transversalement à l'axe longitudinal de la tige (2).

18. Implant pour ostéosynthèse selon l'une quelconque des revendications précédentes, **caractérisé en ce que** les éléments de fixation d'os (7) sont des vis à os.

19. Implant pour ostéosynthèse selon la revendication 18, **caractérisé en ce que** les éléments de fixation d'os (7) présentent au niveau d'une tête de vis (9) un filetage plus grand que dans la partie restante.

20. Implant pour ostéosynthèse selon l'une quelconque des revendications précédentes, **caractérisé en ce que** les ouvertures (6) portent un taraudage.

21. Implant pour ostéosynthèse selon l'une quelconque des revendications précédentes, **caractérisé en ce que** la tige (2) est réalisée de façon droite.

22. Implant pour ostéosynthèse selon l'une quelconque des revendications 1 à 20, **caractérisé en ce que** la tige (2) est courbe au moins par endroits.

23. Implant pour ostéosynthèse selon la revendication 22, **caractérisé en ce que** la courbure (10) de la tige (2) et un coude de la plaque (3) s'étendent dans la même direction.

24. Implant pour ostéosynthèse selon l'une quelconque des revendications précédentes, **caractérisé en ce que** la plaque (3) présente à son extrémité opposée à la tige (2) une zone d'attaque (18) pour un outil de manipulation (11).

25. Implant pour ostéosynthèse selon la revendication 24, **caractérisé en ce que** l'outil de manipulation (11) comprend une pince de serrage (15) à appliquer dans la zone d'attaque (18) et entourant celle-ci par serrage.

26. Implant pour ostéosynthèse selon l'une quelconque des revendications 23 ou 24, **caractérisé en ce que** l'outil de manipulation (11) est relié à un calibre de perçage (21) qui, lorsque l'outil de manipulation (11) est appliqué à la zone d'attaque (18), présente des ouvertures (22) pour un outil de perçage qui sont alignées sur les ouvertures (6 ; 5) dans la plaque (3) et/ou la tige (2).
